(19) Europäisches Patentamt
European Patent Office
Office européen des brevets

(11) EP 0 926 514 A1

(12) DEMANDE DE BREVET EUROPEEN

(43) Date de publication:
30.06.1999 Bulletin 1999/26

(51) Int Cl.6: **G02B 5/22**, A61F 9/02

(21) Numéro de dépôt: **98403216.9**

(22) Date de dépôt: **18.12.1998**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **22.12.1997 FR 9716231**

(71) Demandeur: **AEROSPATIALE**
**75781 Paris Cédex 16 (FR)**

(72) Inventeur: **Adda, Maurice**
**92160 Antony (FR)**

(74) Mandataire: **Joly, Jean-Jacques et al**
**Cabinet Beau de Loménie**
**158, rue de l'Université**
**75340 Paris Cédex 07 (FR)**

# (54) Dispositif de sélection de protections contre les rayonnements laser

(57) Procédé de sélection d'un filtre de protection parmi une pluralité de filtres de protection de caractéristiques optiques prédéterminées, ces filtres étant destinés à protéger le capteur optique d'un récepteur optique vis-à-vis d'une source lumineuse et le récepteur optique et la source lumineuse ayant des caractéristiques optiques prédéterminées, procédé dans lequel on détermine tout d'abord un premier paramètre caractéristique de la portée d'observation du récepteur optique à partir des caractéristiques optiques du récepteur optique, puis on détermine un second paramètre caractéristique de l'éclairement de la source lumineuse à partir des caractéristiques optiques de cette source lumineuse, et en fonction d'un critère de sélection déterminé on compare ces deux paramètres pour chacun des filtres de protection considérés afin d'en sélectionner un particulier. Le premier paramètre est avantageusement une différence (R) entre une portée d'observation calculée sans filtre de protection et une portée d'observation calculée avec filtre de protection et le second paramètre est une densité optique apparente (DOa) obtenue à partir du logarithme en base 10 du rapport entre un éclairement de la source lumineuse au niveau du capteur calculé sans filtre de protection et un éclairement calculé avec un filtre de protection.

L'invention concerne également le dispositif de sélection pour la mise en oeuvre du procédé.

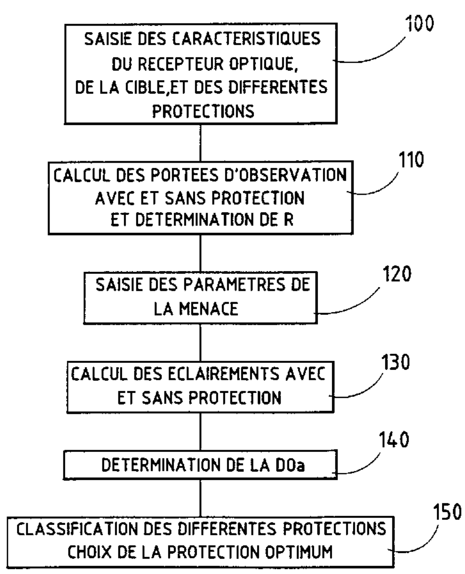


FIG.4

EP 0 926 514 A1

## Description

Domaine de la technique

La présente invention se rapporte au domaine des récepteurs optiques exposés à des rayonnements laser basse énergie et, plus particulièrement, elle concerne un dispositif et un procédé permettant de sélectionner la protection optimale devant être mis en oeuvre pour protéger un capteur optique contre les brouillages ou dommages provoqués par un rayonnement laser.

Art antérieur

Les dispositifs de protection contre les rayonnements laser basse énergie (on parle alors de laser d'éblouissement par opposition au laser destructeur haute énergie) sont bien connus de l'homme de l'art. Ils reposent aussi bien sur des composants passifs (notamment verres organiques à couches colorées) que sur des composants actifs (cristaux liquides à indice de réfraction variable par exemple). Les brevets EP 0 583 048, US 5 561 541 et US 4 737 000 montrent des exemples de réalisation de tels dispositifs dans des domaines aussi bien civil que militaire. Toutefois, la question de leur emploi à bon escient reste posé. En effet, selon les inventeurs, il n'existe à ce jour aucun système qui permette de déterminer, pour un capteur optique et une source laser de caractéristiques données, le dispositif de protection qui, parmi tous les dispositifs envisageables, offre les meilleures performances de détection, de reconnaissance et d'identification pour le capteur optique considéré (qui peut par exemple être l'oeil humain, un détecteur de caméra TV ou encore un détecteur infrarouge).

Définition et objet de l'invention

La présente invention se propose de mettre en oeuvre un procédé permettant de déterminer très simplement le dispositif de protection le mieux adapté à un capteur optique donné soumis à la menace d'une source laser déterminée.

Ces buts sont atteints par un procédé de sélection d'un filtre de protection parmi une pluralité de filtres de protection de caractéristiques optiques prédéterminées, ces filtres étant destinés à protéger le capteur optique d'un récepteur optique vis-à-vis d'une source lumineuse, le récepteur optique et la source lumineuse ayant des caractéristiques optiques prédéterminées, procédé caractérisé en ce qu'on détermine tout d'abord un premier paramètre caractéristique de la portée d'observation du récepteur optique à partir des caractéristiques optiques de ce récepteur optique, puis on détermine un second paramètre caractéristique de l'éclairement de la source lumineuse à partir des caractéristiques optiques de cette source lumineuse, et en fonction d'un critère de sélection déterminé on compare ces deux paramètres pour chacun des filtres de protection considérés afin d'en sélectionner un particulier.

Ainsi, avec ces deux seuls paramètres, il est possible d'obtenir très simplement et rapidement une analyse comparative des performances de différents type de protection et donc d'en déterminer l'intérêt ou non.

Le premier paramètre est de préférence une différence (R) entre une portée d'observation calculée sans filtre de protection et une portée d'observation calculée avec filtre de protection et le second paramètre est une densité optique (DO) obtenue à partir du logarithme en base 10 du rapport entre un éclairement de la source lumineuse au niveau du capteur calculé sans filtre de protection et un éclairement calculé avec un filtre de protection. Avantageusement, les portées d'observation sont déterminées en égalant un contraste minimum résolvable du récepteur optique, intégrant ou non le filtre de protection, avec un contraste apparent d'une cible prédéfinie.

Selon un mode de réalisation préférentiel, la source lumineuse est une source cohérente de type laser.

La présente invention concerne également le dispositif pour la mise en oeuvre de ce procédé.

Brève description des figures

D'autres caractéristiques et avantages de la présente invention ressortiront mieux de la description suivante, faite à titre indicatif et non limitatif, en regard des dessins annexés, sur lesquels:

- la figure 1 montre schématiquement un capteur optique et son environnement,
- la figure 2 illustre un exemple de détermination d'un paramètre du procédé de sélection selon l'invention,
- la figure 3 est un diagramme illustrant les performances des filtres de protection analysés, et
- la figure 4 est un organigramme simplifié de la mise en oeuvre du procédé.

Description détaillée d'un mode de réalisation préférentiel

La figure 1 illustre de façon très schématique un capteur optique dans son environnement de travail. Ce capteur 1 constitue le détecteur optronique (qui peut toutefois être simplement l'oeil de l'opérateur) d'un récepteur optique. Ce récepteur optique comporte classiquement un système optique 2 (appelé aussi voie directe optique) formé, notamment en partie, par l'objectif d'une caméra de télévision, d'une caméra thermique de viseur de tir ou d'un intensificateur d'images. Plus simplement, ce système optique peut être constitué par de simples jumelles d'observation. Le détecteur optronique est soumis à une source lumineuse cohérente 3 dont les caractéristiques basse énergie sont connues de l'opérateur.

Selon l'invention, il est proposé un procédé et un dispositif qui permettent pour un récepteur optique donné et une source lumineuse définie de déterminer le filtre le mieux adapté parmi plusieurs filtres prédéterminés pour protéger le détecteur optronique de ce récepteur optique contre les rayonnements de cette source lumineuse, avantageusement une source cohérente de type laser que l'on identifiera dorénavant par le terme « menace laser ». Ce procédé repose sur la détermination d'une part de portées d'observation avec ou sans filtre et d'autre part d'éclairements avec ou sans filtre et sur la comparaison de ces deux paramètres pour les différents filtres à sélectionner.

On peut caractériser les performances d'un capteur par une portée d'observation que l'on analyse dans ses trois composantes : une portée de détection Pd qui est la distance à partir de laquelle on détecte la présence d'un véhicule porteur de la menace laser (c'est à dire le moment précis où l'opérateur voit un point), une portée de reconnaissance Pr qui est la distance à partir de laquelle cette menace est reconnue et une portée d'identification Pi qui est la distance à partir de laquelle la menace est enfin clairement identifiée (et peut alors être classée parmi un ensemble de menaces pré-identifiées).

Une méthode de calcul de cette portée d'observation est maintenant décrite en regard de la figure 2 qui montre deux courbes représentant les contrastes respectifs de l'image d'une cible théorique (représentant la menace 3) et d'un système optique 2 en fonction de la fréquence spatiale.

Pour la cible (courbe 10), il s'agit d'un contraste apparent qui est fonction du rapport de la luminance dite de « détail » (c'est à dire d'un pixel de l'image) sur la luminance du fond et qui peut s'exprimer selon la relation simplifiée suivante :

$$Ca = (Lp-Lf) / (Lp+Lf) \exp(-\sigma Pob)$$

avec Lp la luminance d'un pixel, Lf la luminance du fond, $\sigma$ un coefficient d'atténuation atmosphérique fonction inverse de la distance de visibilité et Pob la portée d'observation.

Ce contraste apparent est obtenu pour une menace étalon constituée par un char théorique (dite cible OTAN) ayant un volume cubique de 2.30 m de côté. Or, on sait que la fréquence spatiale est le rapport d'un nombre de cycles et d'un angle d'observation et il est donc possible en prenant comme référence les dimensions de ce char théorique d'établir la relation expérimentale suivante entre la fréquence spatiale et la portée d'observation :

$$Fs = 3.5\ Pr / 2.3$$

où le premier coefficient (3.5) correspond par exemple au nombre de cycles nécessaires pour permettre la reconnaissance d'une mire dite « à quatre barres », le second (2.3) correspondant à la largeur de la cible théorique et où Pr est la portée de reconnaissance.

On notera que dans la relation précédente, la portée d'observation est réduite à la seule portée de reconnaissance. Mais, bien entendu, une même détermination du contraste de seuil est aussi envisageable sur la base de la portée d'identification (avec un nombre de cycles différent et supérieur) ou de la portée de détection (avec un nombre de cycles inférieur).

Pour le récepteur optique (courbe 20), le contraste à déterminer est un contraste de seuil ou contraste minimum résolvable (MRC) qui est borné en théorie d'une part par un seuil de visibilité minimale (zone Z1) et d'autre part par une fréquence spatiale limite (zone Z2) et qui en pratique est fonction des dimensions élémentaires du détecteur optronique (ou de la résolution limite de l'oeil) et qui peut s'exprimer par exemple selon la relation simplifiée suivante :

$$Cw = (A\ Fs^2 + B) / MTF$$

où A et B sont des constantes dépendant notamment de l'éclairement rétinien et MTF est la fonction de transfert de modulation du récepteur optique.

Le point de jonction entre ces deux courbes de contraste Ca = f (Fs) et Cw = f (Fs) définit un seuil de non visibilité au delà duquel le capteur ne peut plus observer la menace au travers du système optique et auquel correspond une fréquence spatiale donnée Fso (obtenue par exemple par un calcul itératif à partir des deux formules précédentes) et donc une portée d'observation déterminée.

La présence d'un filtre de protection 4 au niveau du capteur 1 a pour conséquence d'altérer les performances d'observation précitées et donc de réduire les distances à partir desquelles la menace laser peut être observée. Ainsi, les différentes portées correspondant à un capteur protégé prendront des valeurs P'd, P'r et P'i inférieures à celles relatives à un capteur non protégé. Ces valeurs peuvent être calculées, comme précédemment, à partir du contraste de seuil en prenant en compte les caractéristiques optiques du filtre de protection 4 au niveau du système optique 2. La courbe 30 montre la variation de ce contraste de seuil en fonction de la fréquence spatiale et la nouvelle portée d'observation (en l'espèce portée de reconnaissance) ainsi obtenue et elle permet de constater la réduction de portée (R) qui en résulte.

Selon l'invention, à cette détermination d'une portée d'observation avant et après filtrage pour une menace laser et un récepteur optique précisément définis, dont une méthode préférentielle d'obtention a été exposée ci-dessus, il est associé un calcul de l'éclairement laser reçu au niveau du capteur 1 suite à l'émission de la menace laser 3, également avant et après mise en place du filtre de protection 4. Cet éclairement qui est le rapport de la puissance incidente émise par la source lumineuse sur la surface du capteur à l'aire de cette surface peut être déterminé à partir de méthodes matricielles classiques. Des valeurs d'éclairement obtenues au plus près du détecteur, avant (E) et après (Ep) mise en place du filtre de protection, on peut déduire ensuite une densité optique apparente (DOa) qui va caractériser l'atténuation laser de la protection et qui peut être définie par la relation suivante:

$$DOa = Log (E/Ep).$$

Ces deux paramètres que sont la réduction de portée (R) et la densité optique apparente (DOa) sont calculés pour chacun des filtres dont on désire comparer les performances pour un récepteur optique et une menace laser déterminés et le filtre optimum, le mieux adapté à une menace donnée, peut alors être sélectionné selon un critère de sélection déterminé. On peut, par exemple, apprécier la performance du filtre de protection par rapport à un filtre idéal qui aurait une densité optique apparente maximale (pas de perte d'éclairement) et une réduction de portée nulle en positionnant chaque couple (R, DOa) pour un filtre de protection donné dans un plan correspondant.

A partir de ces évaluations de portées et de densités optiques apparentes, il est ainsi possible de se constituer un fichier (une base de connaissance) comportant les filtres les mieux adaptés à différents types prédéterminés de menace. Ce fichier disposé au niveau d'une mémoire 5 (figure 1) accessible par une unité de traitement 6 associée au récepteur optique 1, 2 permettra alors par exemple une mise en place automatique par des moyens de commande 7 du filtre 4 approprié à une menace donnée 3, laquelle peut être identifiée par un dispositif 8 connu en soi d'analyse spectrale des rayonnements reçus.

La figure 3 illustre le cas concret d'un système optique constitué par un viseur de tir ayant les caractéristiques optiques suivantes : grossissement de 8, pupille d'entrée de 50 mm, champ d'observation dans l'oculaire d'environ 50° et coefficient de transmission de l'optique interne de 30%, cette voie étant soumise à un laser émettant une énergie de 1 Joule sur une bande de 532nm avec une divergence de 1 mrd. Des calculs de portée et d'éclairement sont effectués sur quatre types de filtre de protection différents F1 à F4, la réduction de portée et la densité optique apparente en résultant permettant alors de positionner chacun de ces filtres sur le diagramme de cette figure. On en déduit aisément que la meilleure protection envisageable est le filtre F4 qui pour une densité optique apparente sensiblement équivalente au filtre F1 procure une réduction de portée bien moins importante. Le filtre idéal assurant une protection 100% efficace est signalé par la référence F0. Cette détermination graphique est donnée à titre d'illustration pour mieux saisir l'intérêt du procédé selon l'invention. Mais bien entendu, cette sélection du filtre de protection optimum peut être réalisée automatiquement par des méthodes de calcul numériques connues en soi.

Le procédé selon l'invention est résumé de façon schématique par l'organigramme de la figure 4. Dans une première étape 100, on procède à une saisie des caractéristiques optiques du récepteur optique dont on souhaite protéger le détecteur, des paramètres d'une cible théorique et des caractéristiques des filtres de protection à évaluer. A partir de ces éléments, on procède dans une étape 110 à un calcul des différentes portée d'observation avec et sans ces filtres et on en déduit les réductions de portées correspondantes. Dans une étape suivante 120, on saisit les paramètres de la menace laser pour effectuer, dans une étape ultérieure 130, un calcul des éclairements laser avec et sans chacun des filtres de protection, ce calcul étant effectué aux portées d'observations déterminées lors de l'étape 110. Un calcul de la densité optique apparente résultant de la mise en place de ces filtres de protection est ensuite réalisé pour chacun d'entre eux dans une étape 140. L'étape ultime 150 permet une classification des différentes protections et un choix de la meilleure protection par rapport à la menace définie et pour un récepteur optique donné.

On aura compris tout l'intérêt de la présente invention quand on aura noté qu'aujourd'hui on ne sait pas faire le choix, parmi la multiplicité des protections existantes, de la protection à adopter face à une menace pourtant parfaitement identifiée. Tout l'apport de l'invention résulte du fait d'avoir permis d'effectuer ce choix et de l'avoir en outre réalisé de façon particulièrement simple puisqu'il repose sur l'analyse et la comparaison de seulement deux paramètres spécifiques : la portée d'observation et l'éclairement. Bien entendu, différentes méthodes connues de l'homme de l'art sont envisageables pour calculer séparément ces deux paramètres et la méthode explicitée plus avant n'en est qu'un exemple préférentiel. On notera notamment que la méthode précitée est pareillement applicable à une caméra thermique sous réserve de remplacer de calcul du seuil minimum résolvable (MRC) par un calcul d'une différence de température minimum résolvable (MRTD).

**Revendications**

1. Procédé de sélection d'un filtre de protection parmi une pluralité de filtres de protection de caractéristiques optiques prédéterminées, ces filtres (4) étant destinés à protéger le capteur optique (1) d'un récepteur optique vis-à-vis d'une source lumineuse (3) et le récepteur optique et la source lumineuse ayant des caractéristiques optiques prédéterminées, procédé caractérisé en ce qu'on détermine tout d'abord un premier paramètre caractéristique de la portée d'observation du récepteur optique à partir des caractéristiques optiques du récepteur optique, puis on détermine un second paramètre caractéristique de l'éclairement de la source lumineuse à partir des caractéristiques optiques de cette source lumineuse, et en fonction d'un critère de sélection déterminé on compare ces deux paramètres pour chacun des filtres de protection considérés afin d'en sélectionner un particulier.

**2.** Procédé de sélection selon la revendication 1, caractérisé en ce que ledit premier paramètre est une différence (R) entre une portée d'observation calculée sans filtre de protection et une portée d'observation calculée avec filtre de protection.

**3.** Procédé de sélection selon la revendication 2, caractérisé en ce que lesdites portées d'observations sont déterminées en égalant un contraste minimum résolvable du récepteur optique, intégrant ou non le filtre de protection, avec un contraste apparent d'une cible prédéfinie.

**4.** Procédé de sélection selon la revendication 1, caractérisé en ce que ledit second paramètre est une densité optique apparente (DOa) obtenue à partir du logarithme en base 10 du rapport entre un éclairement de la source lumineuse au niveau du capteur calculé sans filtre de protection et un éclairement calculé avec un filtre de protection.

**5.** Procédé de sélection selon la revendication 1, caractérisé en ce que ladite source lumineuse est une source cohérente de type laser.

**6.** Dispositif de sélection d'un filtre de protection parmi une pluralité de filtres de protection de caractéristiques optiques prédéterminées, ces filtres (4) étant destinés à protéger le capteur optique (1) d'un récepteur optique vis-à-vis d'une source lumineuse (3), le récepteur optique et la source lumineuse ayant des caractéristiques optiques prédéterminées, dispositif caractérisé en ce qu'il comporte des moyens (5) de détermination d'un premier paramètre caractéristique de la portée d'observation du récepteur optique à partir des caractéristiques optiques de ce récepteur optique, des moyens (5) de détermination d'un second paramètre caractéristique de l'éclairement de la source lumineuse à partir des caractéristiques optiques de cette source lumineuse, et des moyens (5) de comparaison, en fonction d'un critère de sélection déterminé, de ces deux paramètres pour chacun des filtres de protection considérés afin d'en sélectionner un particulier.

**7.** Dispositif de sélection selon la revendication 6, caractérisé en ce que ladite source lumineuse est une source cohérente de type laser.

FIG.1

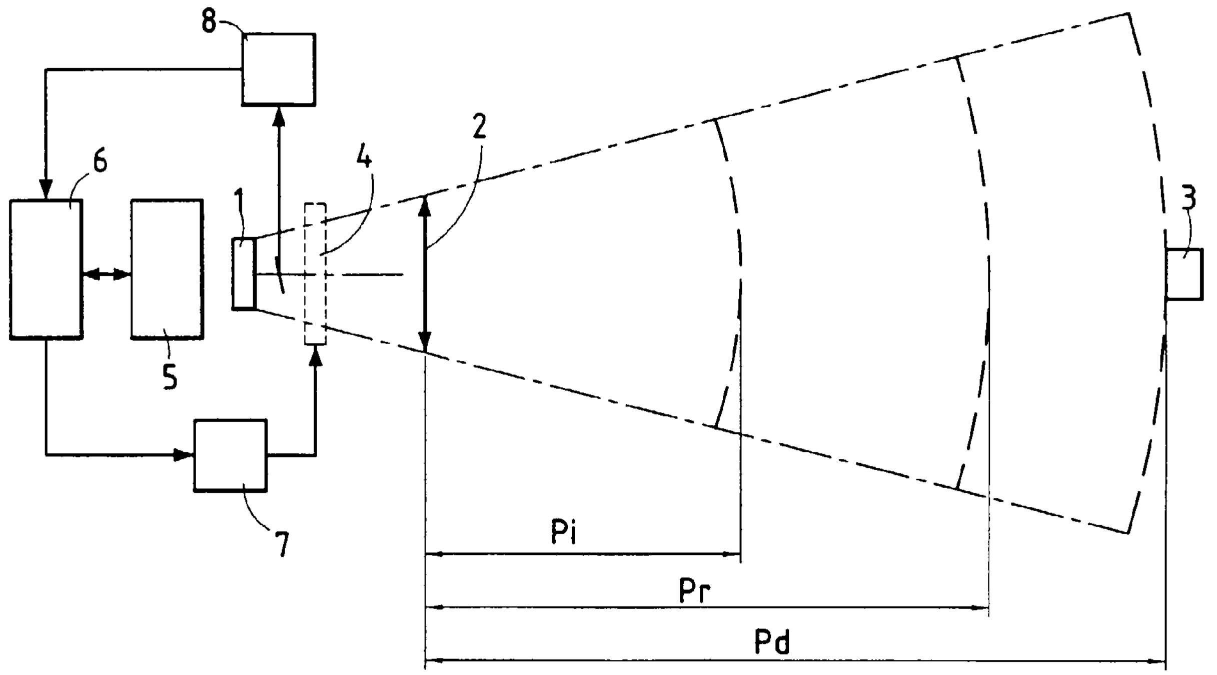


FIG.2

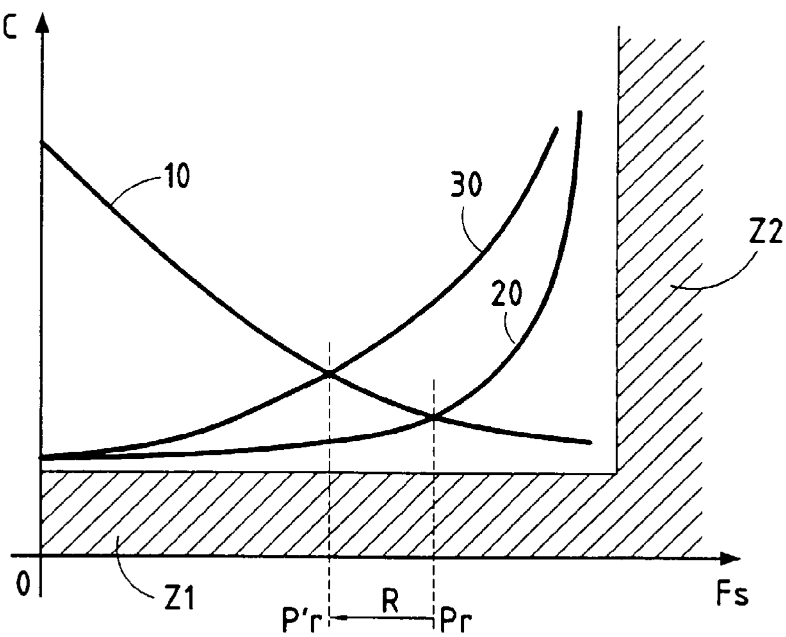

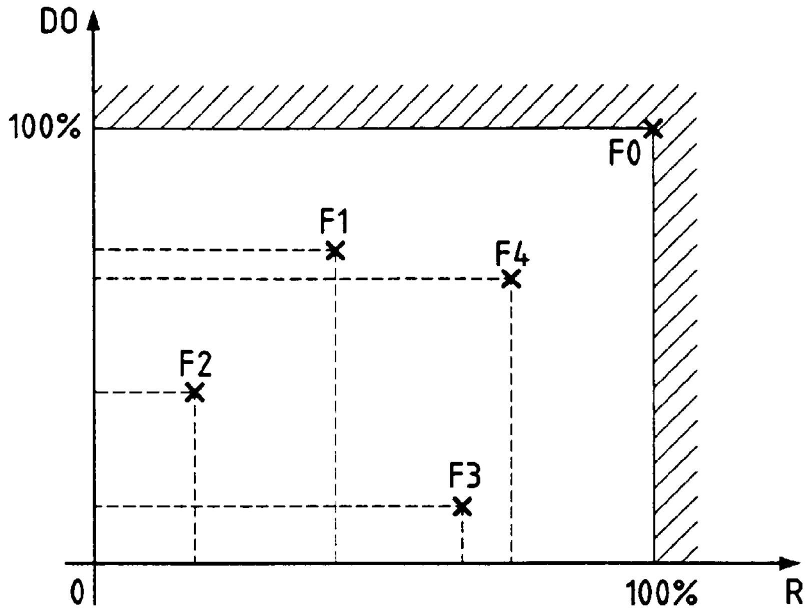
DO
100%
F0
F1
F4
F2
F3
0
100%
R

FIG.3

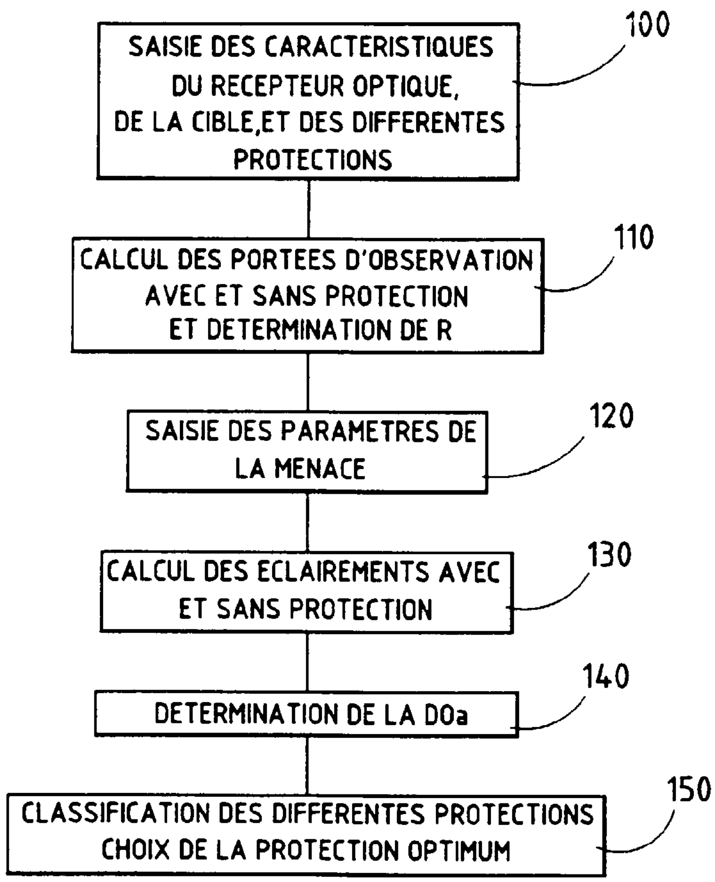
SAISIE DES CARACTERISTIQUES DU RECEPTEUR OPTIQUE, DE LA CIBLE,ET DES DIFFERENTES PROTECTIONS
100
CALCUL DES PORTEES D'OBSERVATION AVEC ET SANS PROTECTION ET DETERMINATION DE R
110
SAISIE DES PARAMETRES DE LA MENACE
120
CALCUL DES ECLAIREMENTS AVEC ET SANS PROTECTION
130
DETERMINATION DE LA DOa
140
CLASSIFICATION DES DIFFERENTES PROTECTIONS CHOIX DE LA PROTECTION OPTIMUM
150

FIG.4

Office européen des brevets

# RAPPORT DE RECHERCHE EUROPEENNE

Numéro de la demande
EP 98 40 3216

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US 4 151 411 A (DERDERIAN GEORGE ET AL) 24 avril 1979<br>* abrégé *<br>* colonne 4, ligne 14 - ligne 33 *<br>* colonne 4, ligne 67 - colonne 5, ligne 31 *<br>* colonne 8, ligne 3 - ligne 27 * | 1-7 | G02B5/22<br>A61F9/02 |
| X | FR 2 572 812 A (SINTRA ALCATEL SA) 9 mai 1986<br>* le document en entier * | 1-4,6 | |
| A | PATENT ABSTRACTS OF JAPAN<br>vol. 008, no. 226 (E-272), 17 octobre 1984<br>& JP 59 105772 A (CANON KK), 19 juin 1984<br>* abrégé * | 1-4,6 | |
| A | PATENT ABSTRACTS OF JAPAN<br>vol. 011, no. 079 (P-555), 11 mars 1987<br>& JP 61 237024 A (FUJI PHOTO FILM CO LTD), 22 octobre 1986<br>* abrégé * | 1-4,6 | DOMAINES TECHNIQUES RECHERCHES (Int.Cl.6)<br>G02B<br>A61F |

Le présent rapport a été établi pour toutes les revendications

| Lieu de la recherche | Date d'achèvement de la recherche | Examinateur |
|---|---|---|
| LA HAYE | 16 avril 1999 | Jakober, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 98 40 3216

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-04-1999

| Document brevet cité au rapport de recherche | Date de publication | Membre(s) de la famille de brevet(s) | Date de publication |
|---|---|---|---|
| US 4151411 A | 24-04-1979 | AUCUN | |
| FR 2572812 A | 09-05-1986 | EP 0182698 A | 28-05-1986 |
| | | FI 854353 A | 07-05-1986 |
| | | JP 61117597 A | 04-06-1986 |
| | | US 4808978 A | 28-02-1989 |

EPO FORM P0460

Pour tout renseignement concernant cette annexe : voir Journal Officiel de l'Office européen des brevets, No.12/82